# EUROPEAN PATENT APPLICATION

(11) **EP 4 534 202 A1**
(43) Date of publication of application: **09.04.2025**
(21) Application number: 22793377.7
(22) Date of filing: 15.07.2022
(51) Int. Cl.: B01L 3/00, C12M 1/34, C12M 1/24, C12M 1/00, C12Q 1/68

(54) **HANDHELD NUCLEIC ACID TESTING DEVICE HAVING MICRO-FLUIDIC CHIP, AND USE METHOD THEREFOR**

(30) Priority: 30.05.2022 CN 202210604648; 30.05.2022 CN 202221327489 U
(71) Applicant: Daan Gene Co., Ltd., Guangzhou, Guangdong 510665 (CN)
(72) Inventor: JIANG, Xiwen, Guangdong 510665 (CN); LIU, Linbo, Guangdong 510665 (CN); SHU, Haitao, Guangdong 510665 (CN); LI, Junjie, Guangdong 510665 (CN)
(74) Representative: Handsome I.P. Ltd
(86) International application number: PCT/CN2022/106129
(87) International publication number: WO 2023/231158

(57) **Abstract**

The present disclosure pertains to the technical field of microfluidic chips, and relates to a hand-held nucleic acid detection apparatus equipped with microfluidic chip, and a use method thereof. The detection apparatus includes a sample collection tube, a chip connector, and a detection chip. The chip connector is provided with a pierce tube configured to pierce the sample collection tube. The detection chip is connected to the chip connector. The detection chip is provided with a plurality of chambers and a plurality of flow channels. The flow channels are in communication with the pierce tube and the chambers. The reaction reagent and all the reaction processes are integrated on a chip, with no need of solution injection by virtue of an external instrument, solution transfer or the like operations, and the nucleic acid detection may be carried out even in the case of no dedicated experiment sites or special conditions. Therefore, the detection apparatus may be applied to communities, hospitals, home self-detection, and public places, such that real-time screening and detection, and self-health state monitoring are achieved during epidemic prevention and control.

## Description

This application is based upon and claims priorities to Chinese Patent Application No. 202210604648.4, filed on May 30, 2022 and entitled "HAND-HELD NUCLEIC ACID DETECTION APPARATUS EQUIPPED WITH MICROFLUIDIC CHIP, AND USE METHOD THEREOF," and Chinese Patent Application No. 202221327489.X, filed on May 30, 2022 and entitled "HAND-HELD NUCLEIC ACID DETECTION APPARATUS EQUIPPED WITH MICROFLUIDIC CHIP," the disclosures of which are incorporated herein by reference in their entireties.

### TECHNICAL FIELD

The present disclosure relates to the technical field of microfluidic chips, and in particular, relates to a hand-held nucleic acid detection apparatus equipped with microfluidic chip, and a use method thereof.

### BACKGROUND

A microfluidic chip, also called a Lab on a Chip, refers to a biological or chemical laboratory built on a chip of a few square centimeters. The basic operation units involved in biological and chemical fields, such as reaction, separation, culture, sorting, and detection, are integrated into a small chip, and micro channels form a network, and a controllable fluid flows throughout the whole system, to implement various functions of a conventional biological or chemical laboratory. The microfluidic chip technology has the characteristics of small sample inlet, high integration, easy automatic control and high-throughput analysis, which makes the biochemical reaction operation on the microfluidic chip more convenient, fast and low-cost relative to the conventional analysis sample pretreatment. The microfluidic technology integrates the complex steps of reagent reaction, separation, and detection on one chip, which not only greatly shortens the sample processing time and greatly reduces the cost of reagents and instruments, but also significantly improves the detection resolution and sensitivity. In addition, the size of the detection apparatus is greatly reduced, making portable and on-site detection possible, and achieving automation and portability of nucleic acid detection.

At present, molecular diagnostic techniques based on nucleic acid detection have been widely used in life, including clinical detection and analysis, food safety, environmental detection, drug screening, and many other fields. Nucleic acid detection procedures generally include the steps of pathogen sampling, sample nucleic acid extraction, PCR amplification, later signal analysis, and result analysis. Traditional nucleic acid detection needs to be completed in the PCR laboratory with professional division. The laboratory needs to be equipped with expensive equipment and instruments, such as a PCR instrument. In addition, a series of operating procedures are cumbersome and complicated, and the detection needs to be carried out by professional personnel. The above-mentioned harsh test conditions result in that only some tertiary hospitals have the qualification and ability to carry out nucleic acid detection at present, and the detection is very complicated and needs professional personnel. In the face of the severe epidemic situation, a nucleic acid self-detection apparatus convenient for users to operate is urgently needed.

### SUMMARY

The present disclosure is to provide a hand-held nucleic acid detection apparatus equipped with a microfluidic chip and a use method thereof, which solve the technical problem that the conventional nucleic acid detection is cumbersome and complicated in operation and needs to be performed by professional personnel.

In order to solve the above problem, embodiments of the present disclosure provide the following technical solution.

A hand-held nucleic acid detection apparatus equipped with a microfluidic chip is provided. The detection apparatus includes:
a sample collection tube;
a chip connector, provided with a pierce tube configured to pierce the sample collection tube; and
a detection chip, connected to the chip connector and provided with a plurality of chambers and a plurality of flow channels, wherein the flow channels are in communication with the pierce tube and the chambers.

Further, the sample collection tube includes a tube body, a sampling solution tube, and a tube cover, wherein the sampling solution tube is arranged in the tube body, the tube cover is rotatably arranged on the tube body, and the tube cover is configured to cover the sampling solution tube.

Further, the detection apparatus further includes: a collection tube top sealing film and a collection tube bottom sealing film, wherein the collection tube top sealing film and the collection tube bottom sealing film are respectively arranged inside the tube cover and at a bottom of the sampling solution tube, and are respectively configured to seal a top and the bottom of the sampling solution tube.

Further, the detection chip includes a first chip and a second chip, wherein the first chip is connected to the second chip, and the chamber is arranged in the first chip.

Further, the sample collection tube further includes a gas buffer tube, arranged in the tube body; and
the flow channels include a sample inlet channel, a fluid outlet channel, a first connection channel, and a second connection channel, wherein the sample inlet channel is in communication with the pierce tube, the fluid outlet channel is in communication with the gas buffer tube, the first connection channel is configured to connect the sample inlet channel to the chambers, and the first connection channel is configured to connect the fluid outlet channel to the chambers.

Further, the chambers are connected in series or in parallel to the channels, and the chambers are pre-loaded with a biochemical reaction reagent, wherein the reagent is a powder, a liquid, a lyosphere, or a solid particle;
the sampling solution tube, the pierce tube, and the sample inlet channel are arranged along a vertical direction, and the reagent in the sampling solution tube flows, through the pierce tube, into a reaction and detection chamber via the sample inlet channel on the detection chip under a gravitational force; and
the flow channels have a width greater than 0 and less than or equal to 10 mm, and the microflow channels on the chip have a depth greater than 0 and less than or equal to 10 mm.

Further, the detection chip further includes a double-sided adhesive film, wherein the first chip and the second chip are connected by the double-sided adhesive film; or
the first chip and the second chip are connected by laser welding, ultrasonic welding, thermocompression bonding, plasma bonding, or solvent bonding.

Further, the detection apparatus further includes a sealing silicone gasket, wherein the sealing silicone gasket is arranged between the sample collection tube and the chip connector and is provided with a first through hole and a second through hole, wherein the first through hole is engaged with the pierce tube, the chip connector is provided with an air outlet in communication with the fluid outlet channel, and the second through hole is engaged with the air outlet.

Further, the air outlet is provided with a hydrophobic air-permeable film, wherein the hydrophobic air-permeable film seals one end, facing towards the chip connector, of the gas buffer tube; and
the hydrophobic air-permeable film has a thickness greater than 0 and less than or equal to 1 mm.

Further, a sealing ring is arranged between the chip connector and the detection chip.

Further, the hydrophobic air-permeable film is made of polytetrafluoroethylene, silicone rubber, or polyethylene; and
the sealing silicone gasket and the sealing ring are made of chloroprene rubber, natural rubber, EPDM rubber, or acrylic rubber.

Further, a latch structure is arranged on an outer side of the chip connector and an inner side of the sample collection tube.

Further, a mounting slot for mounting the detection chip is arranged at an end, facing towards the detection chip, of the chip connector, a first catch slot and a second catch slot are arranged at an end, facing towards the chip connector, of the detection chip, a first catch block engageable with the first catch slot and a second catch block engageable with the second catch slot are arranged at an end, facing towards the detection chip, of the chip connector, the sealing ring is arranged in the first catch slot and attached onto the first catch slot, and the second catch block is provided with an air vent, two ends of the air vent being respectively in communication with the fluid outlet channel and the air outlet.

In order to solve the above problem, embodiments of the present disclosure provide the following technical solution.

A use method of a hand-held nucleic acid detection apparatus equipped with a microfluidic chip is provided. The method is applicable to the detection apparatus as described above. The method includes:
adding a sampling solution of a nucleic acid to be detected into a sample collection tube, and collecting a sample;
mounting a chip connector and a detection chip, and aligning the sample collection tube with the chip connector such that a pierce tube pierces the sample collection tube;
wherein a reaction solution in the sample collection tube flows into a chamber via a flow channel on the detection chip under a gravitational force, and a nucleic acid reaction is carried out between the reaction solution and a pre-loaded reagent in the chamber; and
inserting the detection chip into a companion instrument and acquiring a detection result.

Compared with the related art, the embodiments of the present disclosure mainly achieve the following beneficial effects:

In the hand-held nucleic acid detection apparatus equipped with the microfluidic chip, the reaction reagent and all the reaction processes are integrated on a chip, with no need of solution injection by virtue of an external instrument, solution transfer or the like operations, and the nucleic acid detection may be carried out even in the case of no dedicated experiment sites or special conditions. Specifically, the sampling solution of the nucleic acid to be detected is added into the sample collection tube, the swab cotton head is dipped into the sampling solution tube, and the sample collection tube is aligned with and inserted downwards into the chip connector. Under the effect of the piercing tube, the reaction solution in the sample collection tube flows through the flow channels into the chambers for reaction and detection under the gravitational force, and reacts with the pre-loaded reagent in the chambers, and then the detection chip is inserted into the companion instrument to implement the nucleic acid detection. According to the present disclosure, the operation steps of the nucleic acid detection are greatly simplified, and no strict requirements are imposed onto the operation personnel and the operation sites. In addition, the operation personnel do not need to be dedicatedly trained before carrying out the nucleic acid detection, and thus advantages of simple operation, high detection efficiency, high sensitivity, and the like are achieved. Therefore, the detection apparatus may be applied to communities, hospitals, home self-detection, and public places, such that real-time screening and detection, and self-health state monitoring are achieved during epidemic prevention and control.

### BRIEF DESCRIPTION OF THE DRAWINGS

For clearer descriptions of technical solutions according to the embodiments of the present disclosure, drawings that are to be referred for description of the embodiments are briefly described hereinafter. Apparently, the drawings described hereinafter merely illustrate some embodiments of the present disclosure. Persons of ordinary skill in the art may also derive other drawings based on the drawings described herein without any creative effort.
FIG. 1 is a schematic overall structural view of a hand-held nucleic acid detection apparatus equipped with a microfluidic chip according to some embodiments of the present disclosure;
FIG. 2 is an exploded view of a hand-held nucleic acid detection apparatus equipped with a microfluidic chip according to some embodiments of the present disclosure;
FIG. 3 is a schematic internal structural view of a hand-held nucleic acid detection apparatus equipped with a microfluidic chip according to some embodiments of the present disclosure;
FIG. 4 is a schematic structural view of a sample collection tube according to some embodiments of the present disclosure; and
FIG. 5 is a schematic structural view of a detection chip according to some embodiments of the present disclosure.

Reference numerals and denotations thereof: 1-Sample collection tube; 11-tube body; 12-sampling solution tube; 13-tube cover; 14-gas buffer tube; 2-chip connector; 21-pierce tube; 22-air outlet; 23; mounting slot; 24-first catch block; 25-second catch block; 3-detecting chip; 31-first chip; 32-second chip; 33-chamber; 34-flow channel; 341-sample inlet channel; 342-fluid outlet channel; 343-first connection channel; 344-second connection channel; 35-double-sided adhesive film; 36-first catch slot; 37-second catch slot; 4-collection tube top sealing film; 5-collection tube bottom sealing film; 6-sealing silicone gasket; 61-first through hole; 62-second through hole; 7-hydrophobic air-permeable film; and 8-sealing ring.

### DETAILED DESCRIPTION

Unless otherwise defined, all the technical and scientific terms used in this specification are the same as those usually understood by persons skilled in the art of the present disclosure. Additionally, the terms used in the specification of the present disclosure are merely for description the embodiments of the present disclosure, but are not intended to limit the present disclosure. The terms "comprise," "include," and variations thereof in the specification, claims and accompanying drawings are intended to define a non-exclusive meaning. The terms such as "first," "second," and the like in the specifications, claims or the accompanying drawings of the present invention are intended to distinguishing different objects but are not intended to define a specific sequence.

The term "embodiments" in this specification signifies that the specific feature, structure or feature described with reference to the embodiments may be covered in at least one embodiment of the present disclosure. This term, when appearing in various positions of the specification, neither indicates the same embodiment, nor indicates an independent or optional embodiment that is exclusive of the other embodiments. A person skilled in the art would implicitly or explicitly understand that the embodiments described in this specification may be incorporated with other embodiments.

To make a person skilled in the art better understand the technical solutions of the present disclosure, the technical solutions according to the embodiments of the present invention are clearly and completely described with reference to the accompanying drawings.

### Embodiments

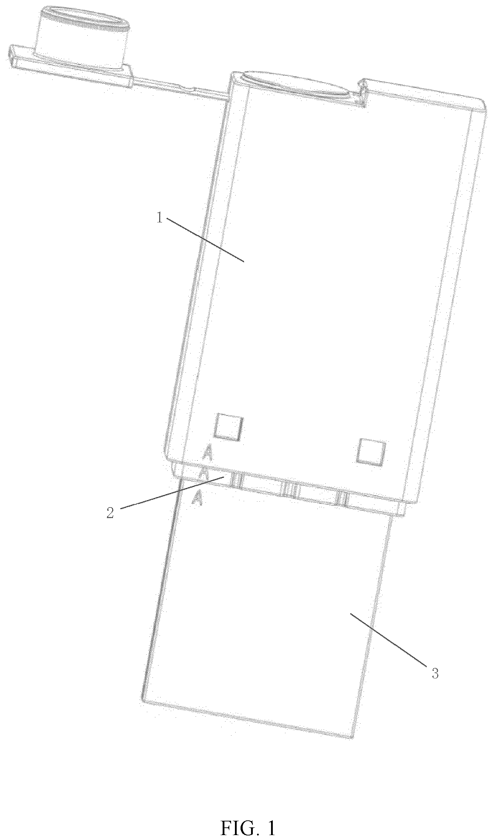

As illustrated in FIG. 1 to FIG. 3, a hand-held nucleic acid detection apparatus equipped with a microfluidic chip includes a sample collection tube 1, a chip connector 2, and a detection chip 3. The chip connector 2 is provided with a pierce tube 21 configured to pierce the sample collection tube 1. The detection chip 3 is connected to the chip connector 2. The detection chip 3 is provided with a plurality of chambers 33 and a plurality of flow channels 34. The flow channels 34 are in communication with the pierce tube 21 and the chambers 33.

In the hand-held nucleic acid detection apparatus equipped with the microfluidic chip, the reaction reagent and all the reaction processes are integrated on a chip, with no need of solution injection by virtue of an external instrument, solution transfer or the like operations, and the nucleic acid detection may be carried out even in the case of no dedicated experiment sites or special conditions. Specifically, the sampling solution of the nucleic acid to be detected is added into the sample collection tube 1, the swab cotton head is dipped into the sampling solution tube 12, and the sample collection tube 1 is aligned with and inserted downwards into the chip connector 2. Under the effect of piercing tube 21, the reaction solution in the sample collection tube 1 flows through the flow channels 34 into the chambers 33 for reaction and detection under the gravitational force, and reacts with the pre-loaded reagent in the chambers 33, and then the detection chip is inserted into the companion instrument to implement the nucleic acid detection. According to the present disclosure, the operation steps of the nucleic acid detection are greatly simplified, and no strict requirements are imposed onto the operation personnel and the operation sites. In addition, the operation personnel do not need to be dedicatedly trained before carrying out the nucleic acid detection, and thus advantages of simple operation, high detection efficiency, high sensitivity, and the like are achieved. Therefore, the detection apparatus may be applied to communities, hospitals, home self-detection, and public places, such that real-time screening and detection, and self-health state monitoring are achieved during epidemic prevention and control.

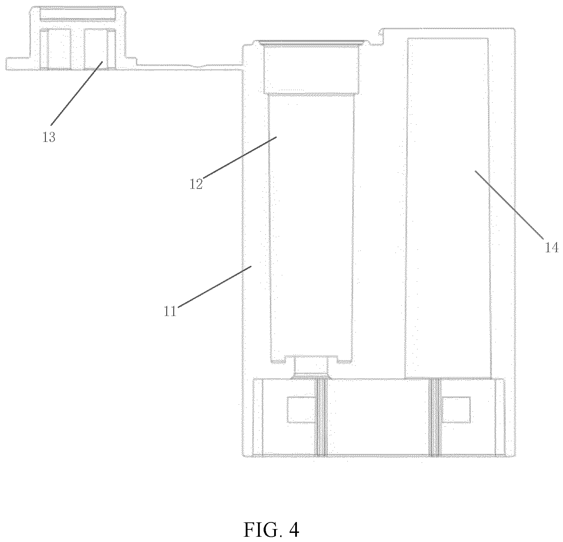

As illustrated in FIG. 1 and FIG. 4, the sample collection tube 1 includes a tube body 11, a sampling solution tube 12, a tube cover 13, and a gas buffer tube 14. The sampling solution tube 12 is arranged in the tube body 11. The tube cover 13 is rotatably arranged on the tube body 11. The tube cover 13 is configured to cover the sampling solution tube 12. The gas buffer tube 14 is arranged in the tube body 11.

In an embodiment, the sampling solution tube 12 contains a lysate, a sampling solution of nucleic acid to be detected is added into the sampling solution tube 12, the tube cover 13 is opened, a swab cotton head is dipped into the sampling solution, rotated and mixed for seconds and broken off into the sampling solution tube 12, and the tube cover 13 is put on. In this way, sample lysis is implemented.

In an embodiment, the sampling solution tube 12 and the gas buffer tube 14 are juxtaposed in the tube body 11.

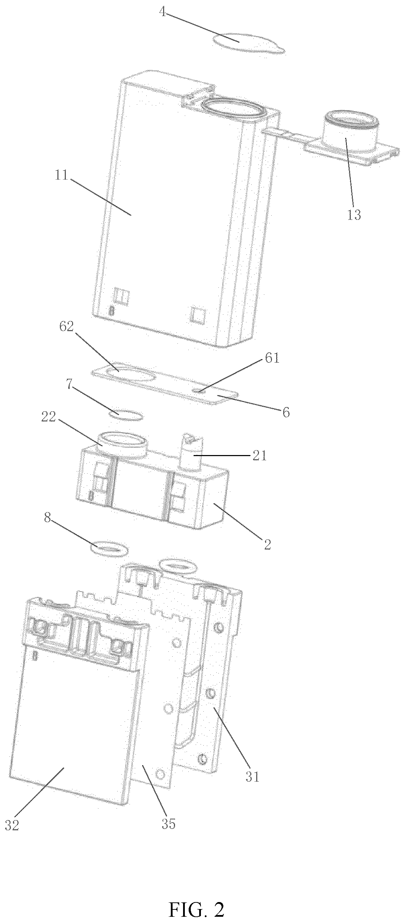

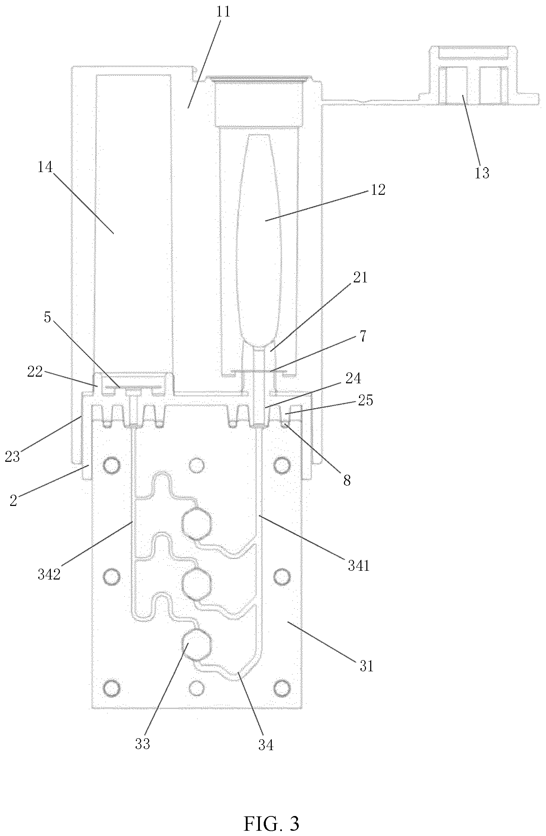

As illustrated in FIG. 2 and FIG. 3, the detection apparatus further includes a collection tube top sealing film 4 and a collection tube bottom sealing film 5, wherein the collection tube top sealing film 4 and the collection tube bottom sealing film 5, and are respectively configured to seal a top and a bottom of the sampling solution tube 12.

In an embodiment, the collection tube top sealing film 4 is prearranged in the tube cover 13, and the collection tube top sealing film 4 seals the tube to prevent the interior of the tube from being in contact with the air and from leakage.

In an embodiment, the collection tube bottom sealing film 5 is prearranged at the bottom of the sample collection tube 1, and the collection tube bottom sealing film 5 prevents the sampling solution from leaking from the sampling solution tube 12.

A sealing ring 8 is arranged between the chip connector 2 and the detection chip 3. Under the effect of the sealing ring 8, a complete sealing is achieved between a lower surface on an inner side of the chip connector 2 and the detection chip 3.

In an embodiment, the sealing ring 8 is an O-shaped sealing ring 8, and the sealing ring 8 is made of chloroprene rubber, natural rubber, EPDM rubber, or acrylic rubber.

As illustrated in FIG. 1 and FIG. 2, the detection chip 3 includes a first chip 31 and a second chip 32, wherein the first chip 31 is connected to the second chip 32, and the chambers 33 are arranged in the first chip 31.

In an embodiment, the first chip 31 is made of glass, a silicon wafer, a polymer, a metal, or a metal oxide.

In an embodiment, the second chip 32 is made of glass, a silicon wafer, a polymer, a metal, or a metal oxide.

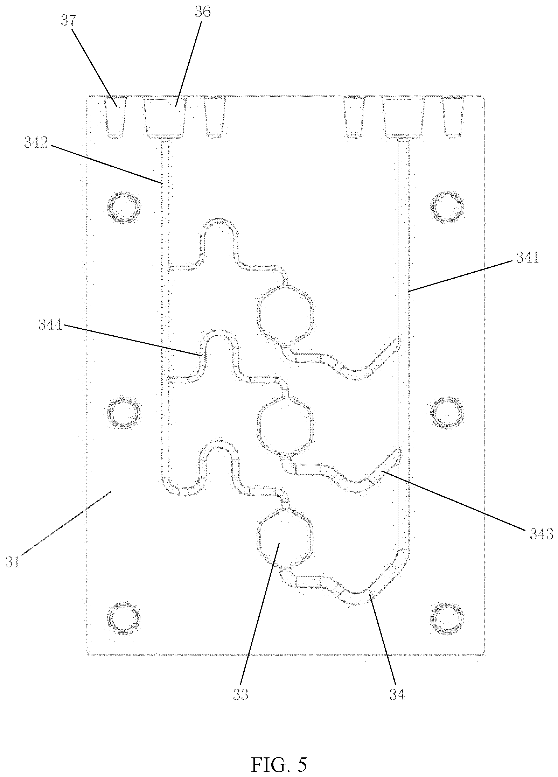

Referring to FIG. 3 and FIG. 5, the flow channels 34 include a sample inlet channel 341, a fluid outlet channel 342, a first connection channel 343, and a second connection channel 344, wherein the sample inlet channel 341 is in communication with the pierce tube 21, the fluid outlet channel 342 is in communication with the gas buffer tube 14, the first connection channel 343 is configured to connect the sample inlet channel 341 to the chambers 33, and the first connection channel 343 is configured to connect the fluid outlet channel 342 to the chambers 33.

In an embodiment, the chambers 33 are connected in series or in parallel to the channels, and the chambers 33 are pre-filled with a biochemical reaction reagent.

In an embodiment, the flow channels 34 are machined by machining over machine tool, laser ablation, 3D printing, injection molding, or chemical etching machining.

In an embodiment, the flow channels 34 are microflow channels, the flow channels 34 have a width greater than 0 and less than or equal to 10 mm, and the microflow channels on the chip have a depth greater than 0 and less than or equal to 10 mm.

In an embodiment, the sampling solution tube 12, the pierce tube 21, and the sample inlet channel 341 are arranged along a vertical direction, and the reagent in the sampling solution tube 12 flows, through the pierce tube 21, into the chambers 33 for reaction and detection via the sample inlet channel 341 on the detection chip 3 under a gravitational force.

The detection apparatus further includes a sealing silicone gasket 6, wherein the sealing silicone gasket 6 is arranged between the sample collection tube 1 and the chip connector 2, and is provided with a first through hole 61 and a second through hole 62, wherein the first through hole 61 is engaged with the pierce tube 21, the chip connector 2 is provided with an air outlet 22 in communication with the fluid outlet channel 342, and the second through hole 62 is engaged with the air outlet 22.

In an embodiment, the first through hole 61 and the second through hole 62 are circular holes or square holes. Specifically, the first through hole 61 and the second through hole 62 are designed to a shape adapted to the pierce tube 21 and the air outlet 22.

In an embodiment, the sealing silicone gasket 6 is made of chloroprene rubber, natural rubber, EPDM rubber, or acrylic rubber.

In an embodiment, an outlet of the fluid outlet channel 342 is aligned with a through hole in a lower end of the gas buffer tube 14 by the chip connector 2 to form a gas communication region.

The air outlet 22 of the chip connector 2 is provided with a hydrophobic air-permeable film 7, wherein the hydrophobic air-permeable film 7 seals one end, facing towards the chip connector, of the gas buffer tube 14, to block passage of liquid.

One opening of the flow channel 34 is a sample inlet, and the other opening of the flow channel 34 extends outside of the detection chip and is connected to the hydrophobic air-permeable film 7. That is, one opening is arranged in the sample inlet channel 341, and the other opening is arranged in the fluid outlet channel 342.

**In** an embodiment, the hydrophobic air-permeable film 7 is made of polytetrafluoroethylene, silicone rubber, or polyethylene.

Preferably, the hydrophobic air-permeable film 7 has a thickness greater than 0 and less than or equal to 1 mm, and the hydrophobic air-permeable film 7 allows passage of the air but blocks passage of liquid.

**In** an embodiment, the chambers 33 are pre-loaded with a biochemical reaction reagent, wherein the reagent is a powder, a liquid, a lyosphere, a solid particle, or the like, and liquid in the sampling solution tube 12 flows into the chambers 33 and reacts with the reagent.

**In** an embodiment, the detection chip 3 further comprises a double-sided adhesive film 35, wherein the first chip 31 and the second chip 32 are connected by the double-sided adhesive film 35.

**In** an embodiment, the first chip 31 and the second chip 32 are connected by laser welding, ultrasonic welding, thermocompression bonding, plasma bonding, or solvent bonding.

A latch structure is arranged on an outer side of the chip connector 2 and an inner side of the sample collection tube 1. In the case that the sample collection tube 1 is inserted downwards into the chip connector 2, the chip connector 2 and the sample collection tube 1 are secured and locked by the latch structure.

A mounting slot 23 for mounting the detection chip 3 is arranged at an end, facing towards the detection chip 3, of the chip connector 2, a first catch slot 36 and a second catch slot 37 are arranged at an end, facing towards the chip connector 2, of the detection chip 3, a first catch block 24 engageable with the first catch slot 36 and a second catch block 25 engageable with the second catch slot 37 are arranged at an end, facing towards the detection chip 3, of the chip connector 2, the sealing ring 8 is arranged in the first catch slot 36 and attached onto the first catch slot 24, and the second catch block 25 is provided with an air vent, two ends of the air vent being respectively in communication with the fluid outlet channel342 and the air outlet 22.

It should be noted that in the case that the hand-held nucleic acid detection apparatus equipped with the microfluidic chip may employ a rectangular cassette shape without departing from the essence of the present disclosure, the detection apparatus may be adjusted according to specific application scenarios, for example, the shape of the detection apparatus may be adjusted according to the machining fashion and functional requirements, and may be specifically a rectangle, cylinder, or a combination of such shapes; the shape of the flow channels 34 on the detection chip 3 may be adjusted according to functional requirements; and the number of chambers 33 on the detection chip 3 may be adjusted according to actual needs.

The detection apparatus according to the embodiments of the present disclosure achieves precise control of sampling addition and detection, is simple to operate, and has a high sensitivity. As such, the detection apparatus may be operated with no dedicated training, achieve a high efficiency, consume fewer samples, achieve quick nucleic acid self-detection, and has an extensive application scope. The detection apparatus may be applied to hospitals, import and export customs, and centers for disease control and prevention, and may also be applied to home self-detection, community detection, outdoor monitoring, and the like nucleic acid detection scenarios.

In order to solve the above problem, embodiments of the present disclosure provide the following technical solution.

A use method of a hand-held nucleic acid detection apparatus equipped with a microfluidic chip is provided. The method is applicable to the detection apparatus as described above. The method includes:
adding a sampling solution of a nucleic acid to be detected into a sample collection tube, and collecting a sample;
mounting a chip connector and a detection chip, and aligning the sample collection tube with the chip connector such that a pierce tube pierces the sample collection tube;
wherein a reaction solution in the sample collection tube flows into a chamber via a flow channel on the detection chip under a gravitational force, and a nucleic acid reaction is carried out between the reaction solution and a pre-loaded reagent in the chamber; and
inserting the detection chip into a companion instrument and acquiring a detection result.

Adding the sampling solution of the nucleic acid to be detected into the sample collection tube includes: adding the sampling solution of the nucleic acid to be detected into the sample collection tube, opening a tube cover, dipping a swab cotton head into the sampling solution, rotating and mixing for seconds and breaking off the cotton swab head into the sampling solution tube, and putting on the tube cover.

In the step of mounting the chip connector and the detection chip and aligning the sample collection tube with the chip connector such that the pierce tube pierces the sample collection tube, the chip connector is inserted into the detection chip, such that the chip connector is fixed and secured to the detection chip, and the chip connector and the detection chip are sealed by the sealing ring; the sample collection tube is aligned with and inserted downwards into the chip connector, the sample collection tube and the chip connector are sealed by the sealing silicone gasket, and the collection tube bottom sealing film is pierced under the effect of piercing.

Inserting the detection chip into the companion instrument and acquiring the detection result include:
inserting the detection chip into the companion instrument, such that the instrument heats the chamber to a set temperature for a nucleic acid amplification reaction; and
detecting, by an optical detection module on the instrument, that an absorbance of the reaction solution changes and a color reaction is carried out, and acquiring the detection result based on a color.

In the use method of the hand-held nucleic acid detection apparatus equipped with the microfluidic chip according to the embodiments of the present disclosure, the reaction reagent and all the reaction processes are integrated on a chip, with no need of solution injection by virtue of an external instrument, solution transfer or the like operations, and the nucleic acid detection may be carried out even in the case of no dedicated experiment sites or special conditions. Specifically, the sampling solution of the nucleic acid to be detected is added into the sample collection tube, the swab cotton head is dipped into the sampling solution tube, and the sample collection tube 1 is aligned with and inserted downwards into the chip connector. Under the effect of piercing, the reaction solution in the sample collection tube flows through the flow channels into the chambers for reaction and detection under the gravitational force, and reacts with the pre-loaded reagent in the chambers, and then the detection chip is inserted into the companion instrument to implement the nucleic acid detection. According to the present disclosure, the operation steps of the nucleic acid detection are greatly simplified, and no strict requirements are imposed onto the operation personnel and the operation sites. In addition, the operation personnel do not need to be dedicatedly trained before carrying out the nucleic acid detection, and thus advantages of simple operation, high detection efficiency, high sensitivity, and the like are achieved. Therefore, the detection apparatus may be applied to communities, hospitals, home self-detection, and public places, such that real-time screening and detection, and self-health state monitoring are achieved during epidemic prevention and control.

It is apparent that the embodiments described above are only exemplary ones, but not all embodiments of the present disclosure, and that the attached drawings illustrate exemplary embodiments of the present disclosure but do not limit the scope of the present disclosure. The present disclosure may be embodied in many different forms and, on the contrary, these embodiments are provided such that the present disclosure are thoroughly and completely understood. Although the present disclosure has been described in detail with reference to the above embodiments, those skilled in the art will be able to make modifications to the technical solutions disclosed in the specific embodiments or make equivalent substitutions for some of the technical features. Any equivalent structure made based on the specification and accompanying drawings of the present disclosure, even if being directly or indirectly applied to some other related technical fields, shall all fall within the protection scope of the present disclosure.

## Claims

1. A hand-held nucleic acid detection apparatus equipped with a microfluidic chip, comprising:
a sample collection tube;
a chip connector, provided with a pierce tube configured to pierce the sample collection tube; and
a detection chip, connected to the chip connector and provided with a plurality of chambers and a plurality of flow channels, wherein the flow channels are in communication with the pierce tube and the chambers.

2. The detection apparatus according to claim 1, wherein
the sample collection tube comprises a tube body, a sampling solution tube, and a tube cover, wherein the sampling solution tube is arranged in the tube body, the tube cover is rotatably arranged on the tube body, and the tube cover is configured to cover the sampling solution tube.

3. The detection apparatus according to claim 2, further comprising:
a collection tube top sealing film and a collection tube bottom sealing film, wherein the collection tube top sealing film and the collection tube bottom sealing film are respectively arranged inside the tube cover and at a bottom of the sampling solution tube, and are respectively configured to seal a top and the bottom of the sampling solution tube.

4. The detection apparatus according to claim 1, wherein
the detection chip comprises a first chip and a second chip, wherein the first chip is connected to the second chip, and the chamber are arranged in the first chip.

5. The detection apparatus according to claim 2, wherein
the sample collection tube further comprises a gas buffer tube, arranged in the tube body; and
the flow channels comprise a sample inlet channel, a fluid outlet channel, a first connection channel, and a second connection channel, wherein the sample inlet channel is in communication with the pierce tube, the fluid outlet channel is in communication with the gas buffer tube, the first connection channel is configured to connect the sample inlet channel to the chambers, and the first connection channel is configured to connect the fluid outlet channel to the chambers.

6. The detection apparatus according to claim 5, wherein
the chambers are connected in series or in parallel to the channels, and the chambers are pre-loaded with a biochemical reaction reagent, wherein the reagent is a powder, a liquid, a lyosphere, or a solid particle;
the sampling solution tube, the pierce tube, and the sample inlet channel are arranged along a vertical direction, and the reagent in the sampling solution tube flows, through the pierce tube, into the chambers for reaction and detection via the sample inlet channel on the detection chip under a gravitational force; and
the flow channels have a width greater than 0 and less than or equal to 10 mm, and the microflow channels on the chip have a depth greater than 0 and less than or equal to 10 mm.

7. The detection apparatus according to claim 4, wherein
the detection chip further comprises a double-sided adhesive film, wherein the first chip and the second chip are connected by the double-sided adhesive film; or
the first chip and the second chip are connected by laser welding, ultrasonic welding, thermocompression bonding, plasma bonding, or solvent bonding.

8. The detection apparatus according to claim 5, further comprising:
a sealing silicone gasket, wherein the sealing silicone gasket is arranged between the sample collection tube and the chip connector and is provided with a first through hole and a second through hole, wherein the first through hole is engaged with the pierce tube, the chip connector is provided with an air outlet in communication with the fluid outlet channel, and the second through hole is engaged with the air outlet.

9. The detection apparatus according to claim 8, wherein
the air outlet is provided with a hydrophobic air-permeable film, wherein the hydrophobic air-permeable film seals one end, facing towards the chip connector, of the gas buffer tube; and
the hydrophobic air-permeable film has a thickness greater than 0 and less than or equal to 1 mm.

10. The detection apparatus according to claim 9, wherein
a sealing ring is arranged between the chip connector and the detection chip.

11. The detection apparatus according to claim 10, wherein
the hydrophobic air-permeable film is made of polytetrafluoroethylene, silicone rubber, or polyethylene; and
the sealing silicone gasket and the sealing ring are made of chloroprene rubber, natural rubber, EPDM rubber, or acrylic rubber.

12. The detection apparatus according to claim 1, wherein
a latch structure is arranged on an outer side of the chip connector and an inner side of the sample collection tube.

13. The detection apparatus according to claim 10, wherein
a mounting slot for mounting the detection chip is arranged at an end, facing towards the detection chip, of the chip connector, a first catch slot and a second catch slot are arranged at an end, facing towards the chip connector, of the detection chip, a first catch block engageable with the first catch slot and a second catch block engageable with the second catch slot are arranged at an end, facing towards the detection chip, of the chip connector, the sealing ring is arranged in the first catch slot and attached onto the first catch slot, and the second catch block is provided with an air vent, two ends of the air vent being respectively in communication with the fluid outlet channel and the air outlet.

14. The detection apparatus according to claim 4, wherein
the first chip is made of glass, a silicon wafer, a polymer, a metal, or a metal oxide; and
the second chip is made of glass, a silicon wafer, a polymer, a metal, or a metal oxide.

15. The detection apparatus according to claim 1, wherein
the flow channels are machined by machining over machine tool, laser ablation, 3D printing, injection molding, or chemical etching machining.

16. The detection apparatus according to claim 10, wherein
the sealing ring is an O-shaped sealing ring, and the sealing ring is made of chloroprene rubber, natural rubber, EPDM rubber, or acrylic rubber.

17. A use method of a hand-held nucleic acid detection apparatus equipped with a microfluidic chip, applicable to the detection apparatus as defined in any one of claims 1 to 16, the method comprising:
adding a sampling solution of a nucleic acid to be detected into a sample collection tube, and collecting a sample;
mounting a chip connector and a detection chip, and aligning the sample collection tube with the chip connector such that a pierce tube pierces the sample collection tube;
wherein a reaction solution in the sample collection tube flows into a chamber via a flow channel on the detection chip under a gravitational force, and a nucleic acid reaction is carried out between the reaction solution and a pre-loaded reagent in the chamber; and
inserting the detection chip into a companion instrument and acquiring a detection result.

18. The method according to claim 17, wherein
adding the sampling solution of the nucleic acid to be detected into the sample collection tube comprises:
adding the sampling solution of the nucleic acid to be detected into the sample collection tube, opening a tube cover, dipping a swab cotton head into the sampling solution, rotating and mixing for seconds and breaking off the cotton swab head into the sampling solution tube, and putting on the tube cover.

19. The method according to claim 17, wherein
mounting the chip connector and the detection chip, and aligning the sample collection tube with the chip connector such that the pierce tube pierces the sample collection tube comprise:
inserting the chip connector into the detection chip, such that the chip connector is fixedly clamped to the detection chip, wherein the chip connector and the detection chip are sealed by a sealing ring; and
inserting downwards the sample collection tube into the chip connector by alignment with the chip connector, wherein the sample collection tube and the chip connector are sealed by a sealing silicone gasket, and under of the effect of the pierce tube, a bottom sealing film of the sample collection tube is pierced.

20. The method according to claim 17, wherein
inserting the detection chip into the companion instrument and acquiring the detection result comprise:
inserting the detection chip into the companion instrument, such that the instrument heats the chamber to a set temperature for a nucleic acid amplification reaction; and
detecting, by an optical detection module on the instrument, that an absorbance of the reaction solution changes and a color reaction is carried out, and acquiring the detection result based on a color.
